# EUROPEAN PATENT APPLICATION

(11) **EP 1 431 291 A1**
(43) Date of publication of application: **23.06.2004**
(21) Application number: 03257833.8
(22) Date of filing: 12.12.2003
(51) Int. Cl.: C07D 231/38, C07D 231/14, C07D 231/16, C07D 231/40, A01N 43/56

(54) **1-Aryl-4-cyano-3-cyclopropylpyrazole derivatives as ectoparasiticidal agents**

(30) Priority: 16.12.2002 US 433841 P
(71) Applicant: Wyeth, Madison, New Jersey 07940 (US)
(72) Inventor: Chiarello, John Francis, Newtown Pennsylvania 18940 (US); Rugg, Douglas, Lebanon New Jersey 08833 (US)
(74) Representative: Connelly, Michael John

(57) **Abstract**

A method and composition for the prevention, amelioration or control of external parasites on animals and humans utilizing a compound of formula I. 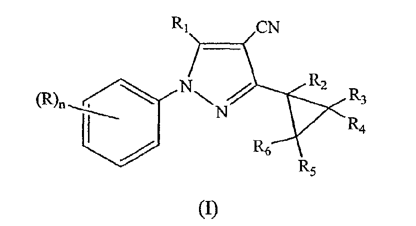 wherein
- R: is halogen, OR₇, SOₘR₈, NO₂, CN, C₁-C₆haloalkyl or an optionally substituted C₁-C₆alkyl group;
- n: is 0 or an integer of 1, 2 or 3;
- m: is 0 or an integer of 1 or 2;
- R₁: is H, halogen, NO₂, NR₉R₁₀, NR₁₁COR₁₂, NCHNR₉R₁₀ or NCHOR₁₃;
- R₂, R₃, R₄, R₅ and R₆: are each independently H, halogen or a C₁-C₄alkyl, aryl or heteroaryl group each optionally substituted;
- R₇: is H or a C₁-C₆alkyl, C₂-C₆alkenyl, C₂-C₆alkynyl, aryl or heteroaryl group each optionally substituted;
- R₈: is a C₁-C₆alkyl, C₂-C₆alkenyl, C₂-C₆alkynyl, aryl or heteroaryl group, each optionally substituted;
- R₉ and R₁₀: are each independently H, C₁-C₄haloalkyl or a C₁-C₆alkyl, C₂-C₆alkenyl, C₂-C₆alkynyl, aryl or heteroaryl group each optionally substituted or R₉ and R₁₀ may be taken together with the atom to which they are attached to form a 5- to 7-membered ring optionally containing 1 or 2 additional heteroatoms selected from O, N or S;
- R₁₁: is H, COR₁₂ or an optionally substituted C₁-C₄alkyl group;
- R₁₂: is a C₁-C₆alkyl, C₂-C₆alkenyl, C₂-C₆alkynyl, aryl or heteroaryl group each optionally substituted; and
- R₁₃: is H or a C₁-C₆alkyl, aryl or heteroaryl group each optionally substituted;
or a stereoisomer or tautomer thereof.

## Description

### FIELD OF THE INVENTION

The present invention is directed to novel compounds and compositions useful in preventing and treating ectoparasiticidal infestation, and more particularly, to new carbonitrile compounds and formulations which are highly efficacious in controlling external parasites in both companion animals and livestock.

### BACKGROUND OF THE INVENTION

Virtually all commercial and most companion animals are affected by ectoparasites. The outcome associated with ectoparasitism in said animals is generally clinical disease and subclinical conditions that decrease performance. Arthropods, such as Phthiraptera (lice) and Diptera (flies), are among the most economically important ectoparasites in animal production. Arthropods, such as Siphonáptera (fleas) and Acarina (ticks) are highly pesteriferous to companion animals and humans and are significant carriers of disease. Ectoparasitic infection and infestation not only seriously effect the economies of raising meat-producing animals but are also a source of great concern for companion animals and humans. New, economic alternative methods and compositions for the prevention, treatment and control of ectoparasites in warm-blooded animals are constantly being sought.

Important agronomic and companion animals such as cattle, sheep, horses, goats, pigs, camels, water buffalo, donkeys, rabbits, fallow deer, reindeer, minks, chinchillas, raccoons, chickens, geese, turkeys, ducks, dogs, cats or the like are prone to attack and infestation by biting and sucking ectoparasitic insects such as Diptera, Muscidae, Acarina or Siphonaptera. In particular, Diptera: Muscidae such as *Musca autumnalis* (face flies), *Haemtobia irritans* (horn flies) *Stomoxys calcitrans* (stable flies), heel flies, tsetse flies, blow flies or the like are breeders of filth and vectors of disease and are serious pests of animals such as cattle, horses and sheep, and, Diptera: Hippoboscidae (louse flies) such as *Melophagus ovinus* (sheep ked), which is a serious parasite of sheep are problematic in animal production.

Acarina, including ticks, e.g., Ixodes spp., *Boophilus microplus, Amblyomma* spp., *Hyalomma* spp., *Rhipicephalus* spp., e.g, *Rhipecephalus sanguineus, Rhipicephalus appendiculatus, Haemaphysalis* spp., *Dermacentor* spp., e.g. *Dermacentor variablis* or the like are serious pests and vectors of disease for man and domestic or companion animals.

Among the Phthiraptera families known to be parasites of animals are: Trichodectidae such as *Bovicola bovis* (important cattle-biting louse), *B. ovis* (sheep-biting louse) or *B. equi* (horsebiting louse); Haematopinidae such as *Haematopinus suis* (hog louse), or *H. asini* (horse sucking louse); Linognathidae such as *Linognathus stenopsis* (goat sucking louse) or *L. vituli* (long-nosed cattle louse); or the like.

Among the Siphonáptera families known to infest companion animals is *Pulicidae* such as Archaeopsyllnìae (cat and dog fleas), Spilopsyllìnae (rabbit fleas), or the like.

The animal health care industry has developed a formidable arsenal of compositions to treat and prevent ectoparasitic infection. Unfortunately, many compounds have lost considerable efficacy over time due to the development of resistant strains of parasites, as well as other factors. What is therefore needed in the art are new compounds and formulations which can be utilized in the ongoing battle to maintain livestock and companion animals free of disease-bearing pests.

### SUMMARY OF THE INVENTION

The present invention provides a compound of formula I 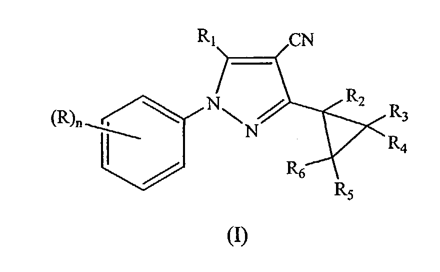 or a pharmaceutically acceptable salt thereof, wherein
- R: is halogen, OR₇, SOₘR₈, NO₂, CN, C₁-C₆haloalkyl or an optionally substituted C₁-C₆alkyl group;
- n: is 0 or an integer of 1, 2 or 3;
- m: is 0 or an integer of 1 or 2;
- R₁: is H, halogen, NO₂, NR₉R₁₀, NR₁₁COR₁₂, NCHNR₉R₁₀ or NCHOR₁₃;
- R₂, R₃, R₄, R₅ and R₆: are each independently H, halogen or a C₁-C₄alkyl, aryl or heteroaryl group each optionally substituted;
- R₇: is H or a C₁-C₆alkyl, C₂-C₆alkenyl, C₂-C₆alkynyl, aryl or heteroaryl group each optionally substituted;
- R₈: is a C₁-C₆alkyl, C₂-C₆alkenyl, C₂-C₆alkynyl, aryl or heteroaryl group each optionally substituted;
- R₉ and R₁₀: are each independently H, C₁-C₄haloalkyl or a C₁-C₆alkyl, C₂-C₆alkenyl, C₂-C₆alkynyl, aryl or heteroaryl group each optionally substituted or R₉ and R₁₀ may be taken together with the atom to which they are attached to form a 5- to 7-membered ring optionally containing 1 or 2 additional heteroatoms selected from O, N or S;
- R₁₁: is H, COR₁₂ or an optionally substituted C₁-C₄alkyl group;
- R₁₂: is a C₁-C₆alkyl, C₂-C₆alkenyl, C₂-C₆alkynyl, aryl or heteroaryl group each optionally substituted (including C₃ - C₇ cycloalkyl); and
- R₁₃: is H or a C₁-C₆alkyl, aryl or heteroaryl group each optionally substituted; and
the stereoisomers and tautomers thereof.

The present invention also provides a compound of formula I as set forth above, but with the further proviso that R₃, R₄, R₅ and R₆ are not all -H, unless R₁ is halogen.

The present invention also provides a method for the prevention, amelioration or control of ectoparasitic infection or infestation in a homeothermic animal which comprises providing to a homeothermic animal in need thereof a prophylactically, therapeutically or pharmaceutically effective amount of a compound of formula I.

This invention also provides a composition which comprises a pharmaceutically acceptable carrier and an ectoparasiticidally effective amount of a compound of formula I.

The invention further provides a composition and method for treating ectoparasitic infection which utilizes the compound of formula I above, with the further proviso that R₃, R₄, R₅ and R₆ are not all -H, unless R₁ is halogen.

It is an object of the present invention to provide compounds which are useful for veterinary medicine and livestock husbandry and in the maintenance of public health against arthropods which are ectoparasites upon homeothermic animals.

It is another object of this invention to provide an effective method for the prevention, treatment or control of ectoparasitic infection or infestation in homeothermic animals.

It is also an object of this invention to provide an ectoparasiticidal composition suitable for use on animals and humans.

Further objects and features of the invention will become apparent from the detailed description set forth herein.

### DETAILED DESCRIPTION OF THE INVENTION

The present invention provides an N-phenyl-3-cyclopropylpyrazole-4-carbonitrile of formula I, above. Unless otherwise specified, "compounds of formula I" includes all the embodiments thereof hereinafter described. Surprisingly, it has now been found that compounds of formula I may be useful in the fields of veterinary medicine and livestock husbandry and in the maintenance of public health against ectoparasites and their infection or infestation.

As used in this specification and the appended claims, the term halogen designates F, Cl, Br or I, and the term heteroaryl designates a C₅-C₁₀ aromatic ring system containing 1, 2 or 3 heteroatoms, which may be the same or different, selected from N, O or S. Such heteroaryl ring systems include pyrrolyl, azolyl, oxazolyl, thiazolyl, imidazolyl, furyl, thienyl, quinolinyl, isoquinolinyl, indolyl, benzothienyl, benzofuranyl, benzisoxazolyl and the like. The term aryl designates a carbocyclic aromatic ring system such as phenyl, naphthyl, anthracenyl or the like. The term haloalkyl as used herein designates a CₙH₂ₙ₊₁ group having from one to 2n+1 halogen atoms which may be the same or different and the term haloalkoxy as used herein designates an OCₙH₂ₙ₊₁ group having from one to 2n+1 halogen atoms which may be the same or different.

In the specification and claims, when the terms C₁-C₆alkyl, C₂-C₆alkenyl, C₂-C₆alkynyl, C₃-C₇cycloalkyl, aryl or heteroaryl are designated as being optionally substituted, the substituent groups which are optionally present may be one or more of those customarily employed in the development of veterinary or pharmaceutical compounds or the modification of such compounds to influence their structure/activity, persistence, absorption, stability or other beneficial property. Specific examples of such substituents include halogen atoms, nitro, cyano, thiocyanato, cyanato, hydroxyl, alkyl, alkanediyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, carbamoyl, alkylamido, phenyl, phenoxy, benzyl, benzyloxy, heterocyclyl or cycloalkyl groups, preferably halogen atoms or lower haloalkyl or lower haloalkoxy groups. Typically, 0-3 substituents may be present, preferably 1 or 2. When any of the foregoing substituents represents or contains an alkyl substituent group, this may be linear or branched and may contain up to 12, preferably up to 6, more preferably up to 4 carbon atoms. Optionally substituted C₁-C₆ alkyl includes methyl substituted by C₂ - C₆ alkanediyl, i.e. includes C₃ - C₇ cycloalkyl.

Compounds of the invention may exist as one or more stereoisomers. The various stereoisomers include enantiomers, diastereomers, atropisomers and geometric isomers. One skilled in the art will appreciate that one stereoisomer may be more active or may exhibit beneficial effects when enriched relative to the other stereoisomer(s) or when separated from the other stereoisomer(s). Additionally, the skilled artisan knows how to separate, enrich or selectively prepare said stereoisomers. Accordingly, the present invention comprises compounds of Formula I, the stereoisomers thereof and the tautomers thereof. The compounds of the invention may be present as a mixture of stereoisomers, individual stereoisomers, or as an optically active or enantiomerically pure form.

Compounds of formula I may exist in one or more tautomeric forms that may give rise to geometric isomers around the tautomeric double bond. One skilled in the art will recognize that said tautomers often exist in equilibrium with one another. As these tautomers interconvert under environmental and physiological conditions, they provide the same useful biological effects. The present invention includes mixtures of such tautomers as well as the individual tautomers of compounds of formula I.

Preferred compounds of the invention include compounds of formula I wherein R is halogen or haloalkyl. Another group of preferred compounds are those formula I compounds wherein R₁ is H, halogen or NR₉R₁₀. A further group of preferred compounds are those of formula I wherein R₅ and R₆ are H.

More preferred compounds of the invention include formula I compounds wherein R is halogen or haloaklyl; n is 3; and R₂ is H, halogen, methyl or optionally substituted phenyl. Another group of more preferred compounds are those of formula I wherein R is Cl or CF₃; n is 3; R₂ is Cl or CH₃; and R₁ is H or Cl.

Especially preferred compounds include those having the formula I set forth above, but with the further proviso that R₃, R₄, R₅ and R₆ are not all -H, unless R₁ is halogen.

Exemplary of some of the preferred compounds of the invention are
5-chloro-3-(2,2-dichloro-1 methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-(2,4,6-trichlorophenyl)-1H-pyrazole-4-carbonitrile;
3-(2,2-dichloro-1,3-dimethylcyclopropyl)-1-[2,6-dichloro-4-(trifluoromethyl)phenyl]-1H-pyrazole-4-carbonitrile;
3-(2,2-dibromo-1-methylcyclopropyl)-1-[2,6-dichloro-4-(trifluoromethyl)phenyl]-1H-pyrazole-4-carbonitrile;
3-(2,2-dibromo-1-methylcyclopropyl)-1-(2,4,6-trichlorophenyl)-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
5-chloro-3-(2,2-dibromo-1-methylcyclopropyl)-1-[2,6-dichloro-4-(trifluoromethyl)phenyl-1H-pyrazole-4-carbonitrile;
5-amino-3-(2,2-dibromo-1-methylcyclopropyl)-1-[2,6-dichloro-4-(trifluoromethyl)phenyl-1 H-pyrazole-4-carbonitrile;
5-bromo-3-(2,2-dibromo-1-methylcyclopropyl)-1-[2,6-dichloro-4-(trifluoromethyl)phenyl]-1H-pyrazole-4-carbonitrile;
5-amino-3-(2,2-dichloro-1-methylcyclopropyl)-1-(2,4,6-trichlorophenyl)-1H-pyrazole-4-carbonitrile;
5-chloro-3-cyclopropyl-1--[2,6-dichloro-4-(trifluoromethyl)phenyl-1 H-pyrazole-4-carbonitrile;
5-chloro-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-5-nitro-1 H-pyrazole-4-carbonitrile;
3-(2,2-dichloro-1-methylcyclopropyl)-1-[2,6-dichloro-4-(trifluoromethyl)phenyl]-5-iodo-1 H-pyrazole-4-carbonitrile;
3-(2,2-dichloro-1-methylcyclopropyl)-1-[2,6-dichloro-4-(trifluoromethyl)phenyl]-5-(dimethylamino)-1 H-pyrazole-4-carbonitrile;
3-(2,2-dichloro-1-methylcyclopyrazol)-1-[2,6-dichloro-4-(trifluoromethyl)phenyl]-5-(diethylamino)-1H-pyrazole-4-carbonitrile;
5-[(cyclopropanecarbonyl)amino-]-3-(2,2-dichloro-1 methylcyclopropyl)-1-[2,6-dichloro-4-(trifluoromethyl)phenyl]-1H-pyrazole-4-carbonitrile;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid methyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid propyl ester;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid ethyl ester;
the stereoisomers thereof or the tautomers thereof.

Compounds of formula I may be prepared using conventional synthetic techniques and, if required, standard separation and isolation techniques. In one preferred embodiment of the method of this invention, compounds of formula I wherein R₁ is NH₂ (la) may be prepared by reacting a phenyl hydrazine of formula II with a cyclopropylcarboxylic acid of formula III to give the intermediate of formula IV; reacting the formula IV intermediate with a chlorinating agent such as thionyl chloride to give the chloroenamine of formula V; and reacting the formula V enamine with malononitrile to give the desired product of formula la. This method is shown in flow diagram I.

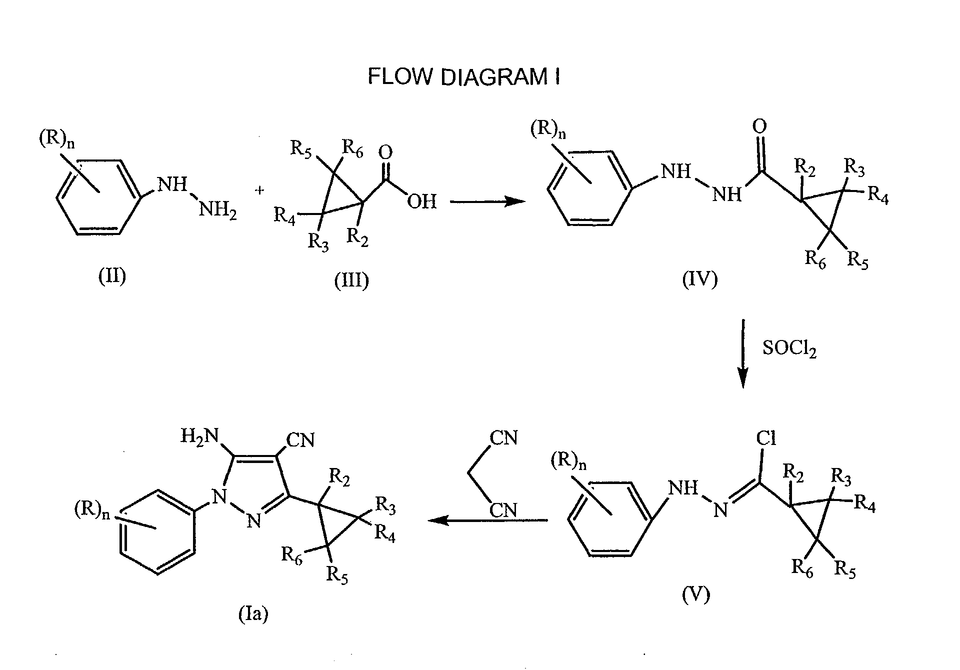

The compounds of formula la may be used as chemical intermediates to prepare the other compounds having formula I using standard derivatization procedures. For instance, the compounds having formula la may be used to prepare the other compounds having formula I in which R₁ is NR₉R₁₀ by standard methods of converting primary amines into secondary amines and tertiary amines, for example, by standard alkylation procedures. In the compounds having formula I in which R₁ is NR₉R₁₀, R₉ and R₁₀ are preferably independently selected from H and C₁-C₆alkyl. The compounds of formula I in which R₁ is NR₉R₁₀ (including the compounds having formula la) may be recovered in the form of their free bases or in the form of their pharmaceutically acceptable acid addition salts. The compounds of formula la and those having formula I in which R₁ is NHR₁₀, R₁₀ being an optionally substituted C₁-C₄alkyl group, may be used to prepare the compounds having formula I in which R₁ is NR₁₁COR₁₂ by standard methods of converting primary amines into amides and imides and secondary amines into amides. Such methods may use reactive derivatives of the carboxylic acid R₁₂CO₂H where R₁₂ is as defined above. R₁₂ is preferably C₁-C₆ alkyl or C₃-C₇ cycloalkyl. Similarly to the aforesaid derivitization, using formula la as substrate and employing the procedures described in US 5,814,652 or Journal of Heterocyclic Chemistry, 2000, 37, 1617, compounds of formula I wherein R₁ is NCHOR₁₃ or NCHNR₉R₁₀, respectively, may be prepared.

The compounds of formula la may be used as chemical intermediates to prepare the compounds having formula I in which R₁ is halogen or nitro via diazonium salts as intermediates. For instance, the compounds having formula I in which R₁ is nitro may be prepared in known manner by treatment of diazonium salts with sodium nitrite in the presence of cuprous ions in neutral or alkaline solution. The compounds having formula I in which R₁ is fluorine may be prepared via diazonium salts by the Balz-Schiemann reaction. The other compounds of formula I wherein R₁ is halogen (Ib) may be prepared by reacting the formula la compound with, in the case wherein the halogen is Cl, concentrated HCl, NaNO₂ and CuCl₂. This reaction is shown in flow diagram II wherein Hal represents Cl, Br or I.

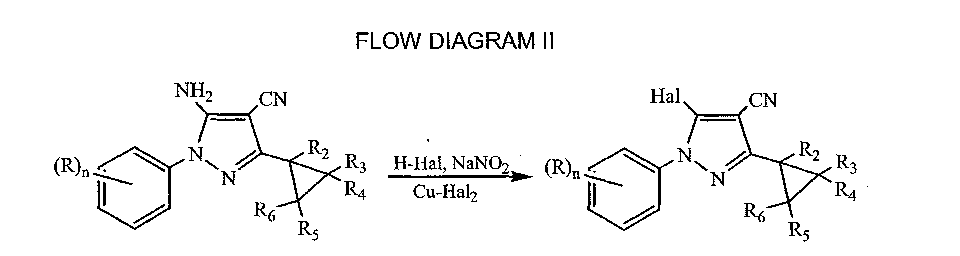

Compounds of formula I wherein R₁ is H may be prepared using conventional deamination techniques such as reacting a compound of formula la with amylnitrite or n-pentylnitrite, as described in EP 303118.

Advantageously, it has now been found that an N-phenyl-3-cyclopropylpyrazole-4-carbonitrile compound of formula I may be used to effectively control, prevent or ameliorate infection and infestation of ectoparasites on homeothermic animals, such as cattle, sheep, horses, goats, pigs, camels, water buffalo, donkeys, rabbits, fallow deer, reindeer, minks, chinchillas, raccoons, chickens, geese, turkeys, ducks, dogs, cats and the like.

Ectoparasites against which a compound of Formula I is useful include biting and sucking ectoparasitic insects such as Diptera, Muscidae, Acarina or Siphonáptera, in particular, Diptera: Muscidae such as *Musca autumnalis* (face flies), *Haemtobia irritans* (horn flies) *Stomoxys calcitrans* (stable flies), heel flies, tsetse flies, blow flies and the like; Diptera: Hippoboscidae (louse flies) such as *Melophagus ovinus* (sheep ked); Acarina, including ticks, e.g., Ixodes spp., *Boophilus microplus, Amblyomma* spp., *Hyalomma* spp., *Rhipicephalus* spp., e.g, *Rhipecephalus sanguineus, Rhipicephalus appendiculatus, Haemaphysalis* spp., *Dermacentor* spp., e.g. *Dermacentor variables*, and the like; the Phthiraptera families, including Trichodectidae such as *Bovicola bovis* (important cattle-biting louse), *B. ovis* (sheep-biting louse), *B. equi* (horsebiting louse), Haematopinidae such as *Haematopinus suis* (hog louse), and *H. asini* (horse sucking louse), Linognathidae such as *Linognathus stenopsis* (goat sucking louse) and *L. vituli* (long-nosed cattle louse), and the like; and the Siphonáptera families, including *Pulicidae* such as Archaeopsyllnìae (cat and dog fleas), Spilopsyllìnae (rabbit fleas), and the like.

Accordingly, the present invention provides a method for the prevention, amelioration or control of ectoparasitic infection or infestation in a homeothermic animal which comprises providing to a homeothermic animal in need thereof a prophylactically, therapeutically or pharmaceutically effective amount of a compound of formula I as described hereinabove.

The term "providing" as used herein with respect to providing a compound or substance embraced by the invention, designates either directly administering or applying such a compound or substance, or administering or applying a prodrug, derivative or analog which forms an equivalent amount of the compound or substance at the locus of administration or application or within the body of the target ectoparasite.

Protection of homeothermic animals from the infestation of ectoparasites, particularly of the orders Diptera, Acarina, Phthiraptera or Siphonáptera may be achieved by the application or administration of a prophylactically, therapeutically or pharmaceutically effective amount of a compound of formula I. In actual practice, the formula I compound may be applied to the animal as a dip, spray, pour-on, spot-on, ear tag, collar, medallion, backrubber, oiler, dustbag, powder, lotion, parenteral injection, or the like, preferably as a topical application such as a spray, pour-on or spot-on.

The effective amount of the formula I N-phenyl-3-cyclopropylpyrazole-4-carbonitrile compound to be used in the method of invention will vary according to the specific compound used, the mode of application used, the identity of the ectoparasite to be controlled, the degree of infestation, the extent of the ectoparasitic insect population, the nature of the target host, the weather conditions or the like. Effective dosages in the practice of this invention typically will range from 0.1 mg/kg to 100 mg/kg, preferably about 1.0 mg/kg to 50 mg/kg based on body weight. Naturally, quantities greater than the effective amounts of the formula I compound may be administered, but are not required for the protection of the target animal from the ectoparasite.

The present invention also provides a veterinary composition which comprises a pharmaceutically acceptable carrier and an ectoparasiticidally effective amount of a compound of formula I. The composition of the invention may be formulated as an aqueous dip for animals such as cattle, sheep, goats or the like or the inventive composition may be prepared as a wettable powder, emulsifiable concentrate, aqueous flowable or the like, which are dispersed in a suitable solvent and applied as sprays to the fur or hide of the animals. Such sprays usually contain about 0.1ppm to 5000ppm and preferably about 0.5ppm to 1000ppm of the active formula I compound. Also preferred is a range of about 0.2 ppm to 20.0 ppm.

Advantageously, the compounds of formula I may also be prepared as pour-on formulations and poured on the backs of the animals such as cattle, sheep or companion animals to protect them against infestation by arthropod ectoparasites. The pour-on compositions of the invention are generally prepared by dissolving, suspending or emulsifying the formula I compound in a suitable nontoxic pharmaceutically acceptable diluent for pour-on administration. The diluent must be compatible with the ectoparasiticidal compound and should not be a source of irritation or damage to the animal's skin or hair. Such diluents include mono and polyhydric alcohols, vegetable oils, spreading oils, aliphatic and aromatic hydrocarbons, lower alkyl ketones, esters and fatty acids; such diluents are well-known in the art. Those skilled in the art will readily be able to identify suitable diluents for use in this invention.

In one embodiment of this invention, a pour-on formulation comprises about 0.5% to 30% by weight of the compound of formula I, about 0.5 to 30% by weight of a spreading oil, 30% to 60% by weight of an aliphatic or aromatic hydrocarbon, mono or polyhydric alcohol, lower alkyl ketone or mixtures thereof and 0% to 20% by weight of a vegetable or mineral oil. A more preferred embodiment thereof comprises about 10% to 25% by weight of the compound of formula I, about 20 to 30% by weight of a spreading oil, 40% to 50% by weight of an aliphatic or aromatic hydrocarbon, mono or polyhydric alcohol, lower alkyl ketone or mixtures thereof and 5% to 15% by weight of a vegetable or mineral oil.

In another embodiment of this invention, a pour-on formulation comprises 45% by weight of xylene, 25% by weight of cyclohexanone, 15% by weight of said antagonist compound, 10% by weight of corn oil or mineral oil and 5% by weight of other pharmaceutically acceptable diluents such as surfactants, spreading agent, antifoam agents or the like.

Among the spreading oils that can be utilized in pour-on formulations of this invention are fatty acids, fatty acid esters, triglycerides and fatty alcohols including: isopropyl myristate, capryl/caproic acid esters of saturated (C₁₂ -C₁₈) fatty alcohols with waxy fatty acid esters, isopropyl palmitate and the like.

Useful alcohols, glycols and ketones in the practice of this invention include: ethyl alcohol, isopropyl alcohol, propylene glycol, dipropylene glycol, benzyl alcohol, dipropylene glycol monoethyl ether, cyclohexanone, methylethyl ketone, methylisobutyl ketone, N-butoxybutylethoxyethanol and the like. Also, the vegetable oils that may be utilized in these formulations include: corn oil, olive oil, peanut oil, sunflower oil, cottonseed oil, soybean oil, and the like. Hydrocarbons that can be used in the formulation of this invention include: xylene, toluene, and the like.

Surfactants may also be utilized in the formulations if desired. Those skilled in the art will readily be able to determine which surfactants known in the art will be suitable in the practice of this invention, and will understand how to incorporate such surfactants into formulations of this invention.

In order to present a more clear understanding of the invention, the following specific examples are set forth below. These examples are merely illustrative and are not to be understood as limiting the scope and underlying principles of the invention in any way. Indeed, various modifications of the invention, in addition to those illustrated and described herein, will become apparent to persons skilled in the art from the following examples and the foregoing description. Such modifications are also intended to fall within the scope of the appended claims. The terms HNMR and THF designate proton nuclear magnetic resonance and tetrahydrofuran, respectively. Unless otherwise noted, all parts are parts by weight.

### EXAMPLE 1

### PREPARATION OF 2,2-DICHLORO-1-METHYLCYCLOPROPYL CARBOXYLIC ACID, N'-[2,6-DICHLORO-4-(TRIFLUOROMETHYL)PHENYL]HYDRAZIDE

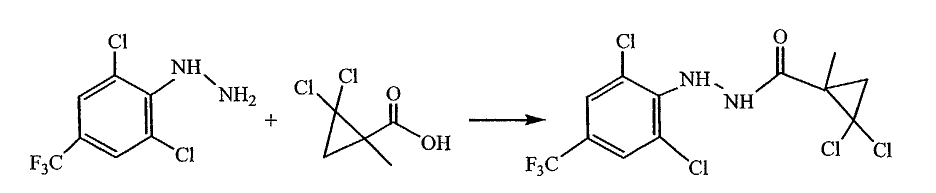

A mixture of 2,6-dichloro-4-(trifluoromethyl)phenyl hydrazine (25 g, 102 mmol) and 2,2-dichloro-1-methylcyclopropylcarboxylic acid (17 g, 102 mmol) in CH₂Cl₂ is treated portion-wise over a 15 min. period with 1-[3-(dimethylamino)propyl]-3-ethylcarbodiimide hydrochloride (20 g, 102 mmol), stirred at room temperature for 18h, diluted with water, stirred for 0.5h and filtered. SThe filtercake is dried, washed with 1:1 mixture of ether:hexanes and dried *in vacuo* to afford the title product as a white solid, 33 g (82% yield), mp 174-175°C, identified by HNMR and mass spectral analyses.

### EXAMPLE 2

### PREPARATION OF N1-[2,6-DICHLORO-4-(TRIFLUOROMETHYL)PHENYL]-(2,2-DICHLORO-1-METHYLCYCLOPROPANE)-1-CARBOHYDRAZONOYL CHLORIDE

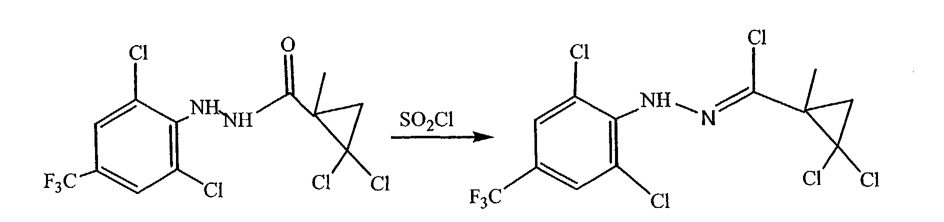

A stirred mixture of 2,2-dichloro-1-methylcyclopropyl carboxylic acid, N'-[2,6-dichloro-4-(trifluoromethyl)phenyl]hydrazide (9.4 g, 23.7 mmol) in toluene is treated dropwise with thionyl chloride (7.0 mL, 95 mmol), heated at reflux temperature for 3h, cooled to room temperature and concentrated *in vacuo*. The resultant oil residue is purified through a short bed of silica gel using hexanes as eluent to afford the title product as a tan solid, 9.1 g (93% yield), identified by HNMR and mass spectral analyses.

### EXAMPLE 3

### PREPARATION OF 5-AMINO-3-(2,2-DICHLORO-1-METHYLCYCLOPROPYL)-1-[2,6-DICHLORO-4-(TRIFLUOROMETHYL)PHENYL]-1H-PYRAZOLE-4-CARBONITRILE

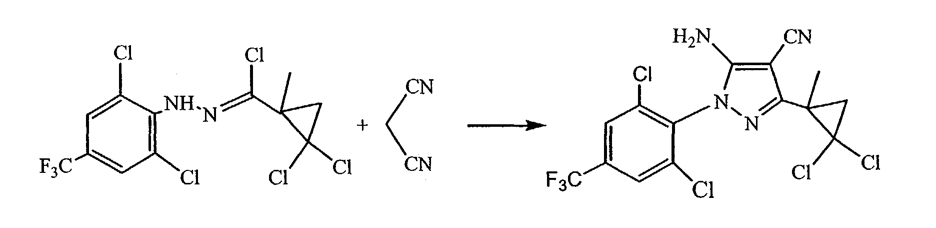

A solution of malonitrile (7.23 mL, 115 mmol) in THF is treated with NaH

(2.30 g, 57.5 mmol), cooled to 0°C, treated with a solution of [Ex. 2] (9.10 g, 23 mmol) in THF over a 0.5h period, stirred at 0°C for 1 h, warmed to 20°C, stirred for 0.5h and diluted with water and ether. The phases are separated. The organic phase is washed with brine, dried over Na₂SO₄ and concentrated *in vacuo*. The resultant residue is purified by flash chromatography (silica gel, 40% ether in hexanes as eluent) to afford the title product as a white solid, 6.15 g (60% yield), mp >200°C, identified by HNMR and mass spectral analyses.

### EXAMPLE 4

### PREPARATION OF 5-CHLORO-3-(2,2-DICHLORO-1-METHYLCYCLOPROPYL)-1-[2,6-DICHLORO-4-(TRIFLUOROMETHYL)PHENYL]-1H-PYRAZOLE-4-CARBONITRILE

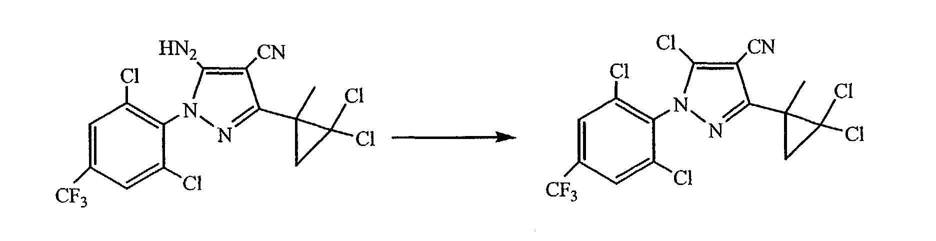

A mixture of 5-Amino-3-(2,2-dichloro-1-methylcyclopropyl)-1-[2,6-dichloro-4-(trifluoromethyl)phenyl]-1H-pyrazole-4-carbonitrile (5.00 g, 11.3 mmol) in concentrated HCl (30 mL) at 0°C is treated portion-wise with NaNO₂ (7.80 g, 113 mmol) over a 15 min. period, stirred for 1 h at 0°C, treated with CuCl₂ (2.24 g, 22.6 mmol), heated slowly to 55°C, stirred at 55°C for 1h, cooled to room temperature and decanted. The remaining solid is dispersed in a mixture of water and ether. The phases are separated. The organic phase is dried over Na₂SO₄ and concentrated *in vacuo.* The resultant residue is purified by flash chromatography to afford the title product as a red resin, 3.40 g (65% yield), identified by HNMR and mass spectral analyses.

### EXAMPLES 5-40

### PREPARATION OF 3-CYCLOPROPYL-1-PHENYLPYRAZOLE-4-CARBONITRILE DERIVATIVES

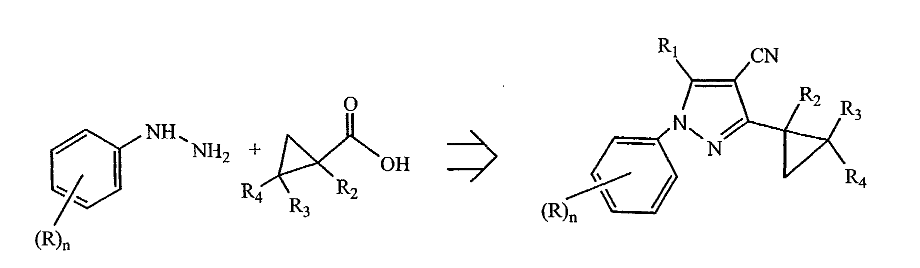

Using essentially the same procedures described in the foregoing Examples 1-4 and employing the appropriate hydrazine and cyclopropylcarboxylic acid, the compounds shown in Table I are obtained and identified by HNMR and mass spectral analyses.

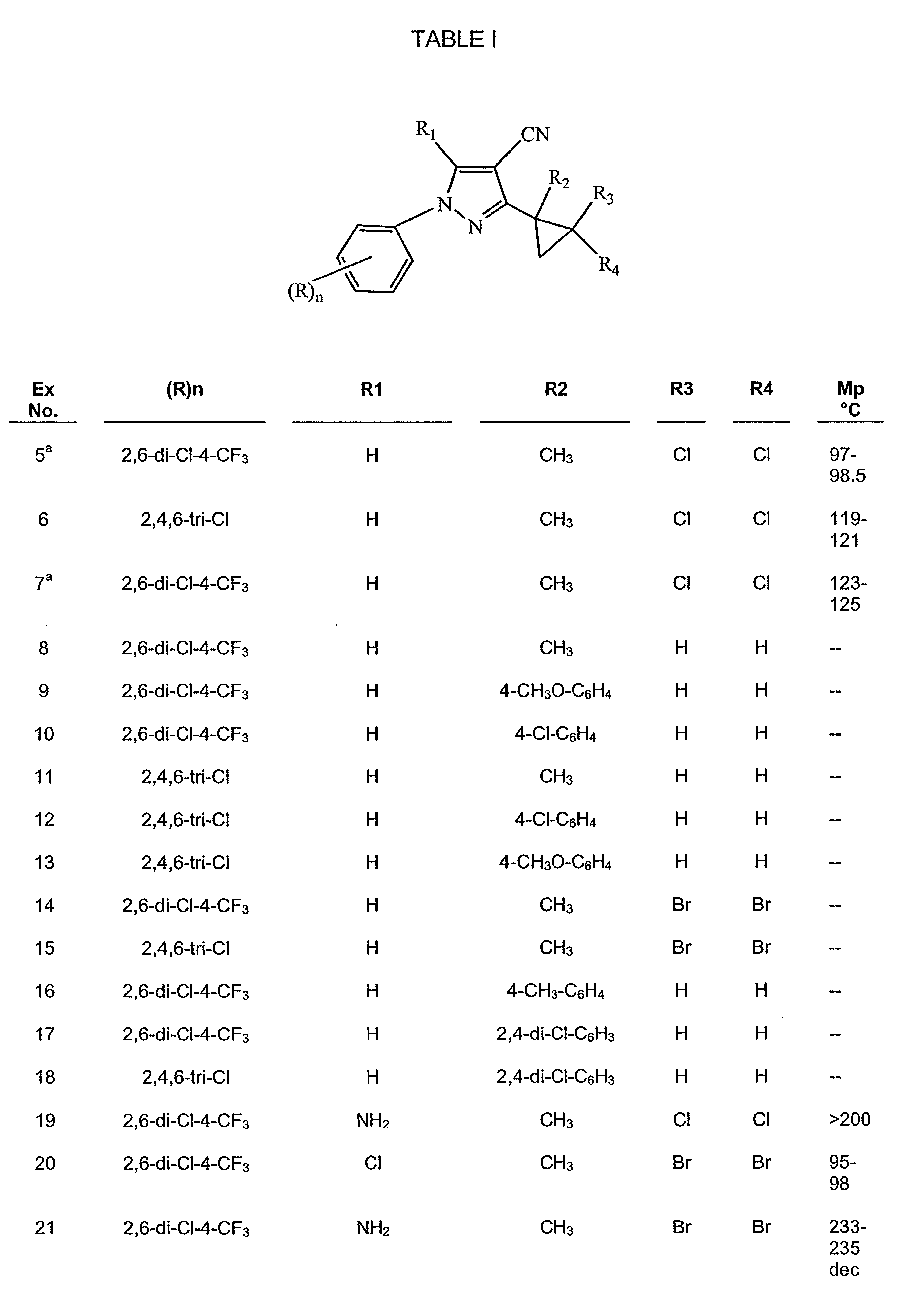

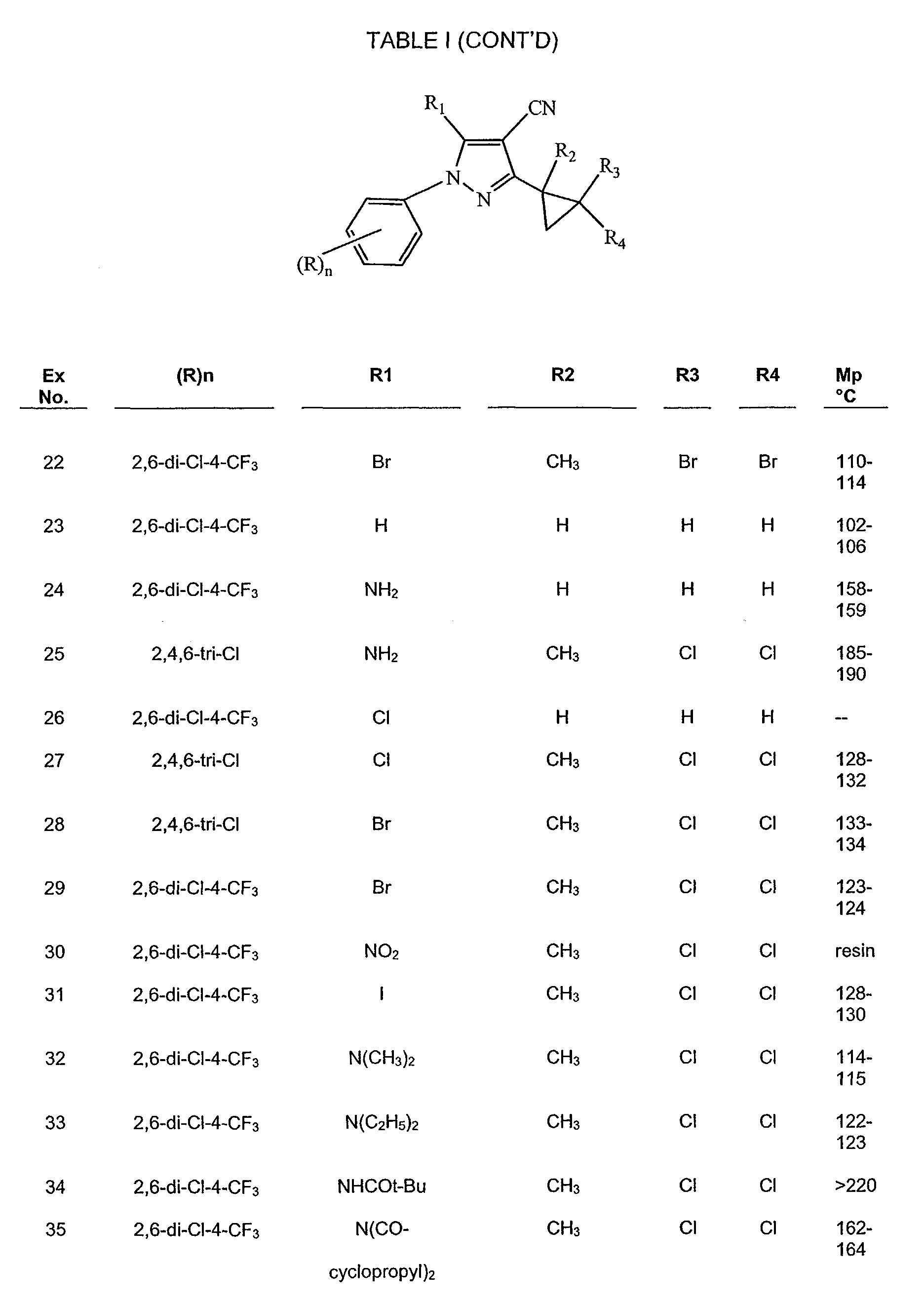

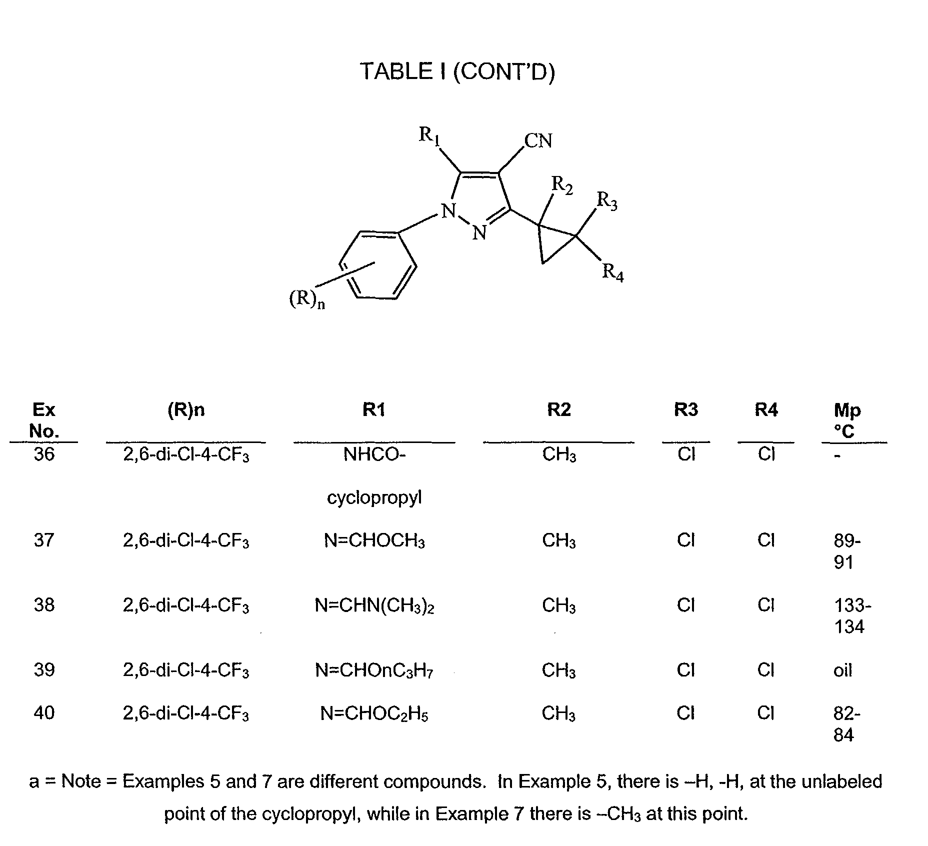

### EXAMPLE 41

### EVALUATION OF THE EFFICACY OF TEST COMPOUNDS AGAINST ADULT CAT FLEAS

The contact activity of a test compound against *Ctenocephatides felis*, adult cat fleas, is evaluated in a coated, glass vial assay. In this evaluation, a solution of test compound in acetone is allowed to dry on the interior surface of a 20 mL vial. Ten unfed adult cat fleas are added to the treated vial and mortality is assessed periodically for up to 48h. All treatments are replicated 2 times. The data are averaged. The results are shown in Table II.

**TABLE II**

| **Test** **Compound** **(Ex. No.)** | **Conc.** **(ug/cm2)** | **% Mortality** | |
|---|---|---|---|
| | | **4h** | **24h** |
| 4 | 2 | 100 | 100 |
| 4 | 0.2 | 60 | 100 |
| 4 | 0.002 | 20 | 100 |
| 5 | 2 | 0 | 33 |
| 7 | 2 | 0 | 100 |
| 14 | 2 | 10 | 100 |
| 15 | 2 | 10 | 80 |
| 19 | 2 | 0 | 10 |
| 20 | 2 | 20 | 100 |
| 21 | 2 | 0 | 0 |
| 22 | 2 | 0 | 100 |
| 26 | 2 | 100 | 100 |
| 27 | 2 | 10 | 100 |
| 28 | 2 | 0 | 10 |
| 29 | 2 | 50 | 100 |
| 29 | 0.2 | 20 | 100 |
| 29 | 0.02 | 10 | 100 |
| 30 | 2 | 0 | 100 |
| 31 | 2 | 20 | 100 |
| 32 | 2 | 0 | 100 |
| 33 | 2 | 20 | 100 |
| 37 | 2 | 10 | 100 |
| 39 | 2 | 0 | 70 |
| 40 | 2 | 0 | 100 |
| Standard¹ | 2 | 30 | 100 |
| Standard¹ | 0.2 | 10 | 100 |
| Standard¹ | 0.02 | 0 | 100 |
| Control² | 0 | 0 | 0 |

| | | | |
|---|---|---|---|
| ¹Fipronil | | | |
| ²Untreated | | | |

### EXAMPLE 42

### EVALUATION OF THE EFFICACY OF TEST COMPOUNDS AGAINST ADULT TICKS

The contact activity of a test compound against *Rhipacephalus sanguineus*, adult ticks, is evaluated in a coated glass vial assay. In this evaluation, a solution of test compound in acetone is allowed to dry on the interior surface of a 20 mL vial. Five unfed adult ticks are added to the treated vial and mortality is assessed periodically for up to 48h. All treatments are replicated 2 times. The data are averaged. The results are shown in Table III.

**Table III**

| **Test** **Compound** **(Ex. No.)** | **Conc.** **(ug/cm2)** | **% Mortality** | |
|---|---|---|---|
| | | **4h** | **24h** |
| 4 | 7.80 | 60 | 100 |
| 4 | 0.78 | 60 | 100 |
| 7 | 7.80 | 10 | 80 |
| 7 | 0.78 | 0 | 10 |
| 14 | 7.80 | 50 | 80 |
| 14 | 0.78 | 40 | 40 |
| 19 | 7.80 | 40 | 70 |
| 19 | 0.78 | 0 | 20 |
| 20 | 7.80 | 20 | 50 |
| 20 | 0.78 | 0 | 20 |
| 21 | 7.80 | 0 | 40 |
| 21 | 0.78 | 10 | 10 |
| 22 | 7.80 | 0 | 60 |
| 22 | 0.78 | 20 | 30 |
| 29 | 7.80 | 20 | 60 |
| 29 | 0.78 | 0 | 40 |
| 31 | 7.80 | 50 | 50 |
| 31 | 0.78 | 10 | 20 |
| 32 | 7.80 | 0 | 30 |
| 32 | 0.78 | 0 | 60 |
| Standard¹ | 7.80 | 100 | 100 |
| Standard¹ | 0.78 | 90 | 100 |
| Control² | 0 | 0 | 3 |

| | | | |
|---|---|---|---|
| ¹Fipronil | | | |
| ²Untreated | | | |

### EXAMPLE 43

### EVALUATION OF EFFICACY OF TEST COMPOUNDS AGAINST BLOWFLY LARVAE

The efficacy of a test compound against newly emerged larvae of *Lucilia sericata*, blowfly, is evaluated using a treated paper/serum bioassay. In this evaluation, a solution of test compound in acetone is applied to a filter paper disc and the disc is allowed to dry. Bovine serum and about 20 newly emerged blowfly larvae are added to the treated paper disc and the mortality is assessed at 24h and 48h. The data are averaged. The results are shown in Table IV.

**TABLE IV**

| **Test** **Compound** **(Ex. No.)** | **Conc.** **(ppm)** | **% Mortality** | |
|---|---|---|---|
| | | **24h** | **48h** |
| 4 | 20.0 | 100 | 100 |
| 4 | 2.0 | 100 | 100 |
| 4 | 0.2 | 100 | 100 |
| 6 | 20.0 | 70 | 70 |
| 6 | 1.0 | 10 | 10 |
| 7 | 20.0 | 77 | 90 |
| 7 | 2.0 | 0 | 0 |
| 9 | 20.0 | 0 | 0 |
| 10 | 20.0 | 0 | 0 |
| 11 | 20.0 | 0 | 0 |
| 13 | 20.0 | 0 | 0 |
| 14 | 20.0 | 95 | 100 |
| 14 | 2.0 | 100 | 100 |
| 14 | 0.2 | 50 | 50 |
| 15 | 20.0 | 90 | 90 |
| 15 | 2.0 | 95 | 95 |
| 15 | 0.2 | 0 | 0 |
| 17 | 20.0 | 0 | 0 |
| 18 | 20.0 | 0 | 0 |
| 19 | 20.0 | 75 | 95 |
| 19 | 2.0 | 100 | 100 |
| 19 | 0.2 | 0 | 0 |
| 20 | 20.0 | 100 | 100 |
| 20 | 2.0 | 100 | 100 |
| 20 | 0.2 | 0 | 0 |
| 21 | 20.0 | 90 | 100 |
| 21 | 2.0 | 95 | 100 |
| 21 | 0.2 | 0 | 0 |
| 22 | 20.0 | 100 | 100 |
| 22 | 2.0 | 100 | 100 |
| 22 | 0.2 | 0 | 0 |
| 24 | 10.0 | 0 | 0 |
| 25 | 20.0 | 5 | 5 |
| 25 | 10.0 | 0 | 0 |
| 26 | 20.0 | 97 | 97 |
| 26 | 10.0 | 0 | 50 |
| 26 | 2.0 | 0 | 0 |
| 27 | 20.0 | 100 | 100 |
| 27 | 2.0 | 100 | 100 |
| 27 | 0.2 | 0 | 0 |
| 28 | 20.0 | 100 | 100 |
| 28 | 2.0 | 95 | 100 |
| 28 | 0.2 | 0 | 0 |
| 29 | 20.0 | 100 | 100 |
| 29 | 2.0 | 100 | 100 |
| 29 | 0.2 | 15 | 15 |
| 30 | 20.0 | 20 | 20 |
| 30 | 10.0 | 10 | 50 |
| 31 | 20.0 | 97 | 100 |
| 31 | 2.0 | 95 | 100 |
| 31 | 0.2 | 0 | 0 |
| 32 | 20.0 | 91 | 96 |
| 32 | 2.0 | 0 | 0 |
| 32 | 0.2 | 0 | 0 |
| 33 | 20.0 | 100 | 100 |
| 33 | 2.0 | 90 | 90 |
| 33 | 0.2 | 0 | 0 |
| 34 | 20.0 | 0 | 0 |
| 35 | 20.0 | 0 | 0 |
| 36 | 20.0 | 10 | 10 |
| 36 | 10.0 | 0 | -- |
| 37 | 20.0 | 100 | 100 |
| 37 | 2.0 | 0 | 0 |
| 38 | 20.0 | 0 | 0 |
| 39 | 20.0 | 100 | 100 |
| 39 | 2.0 | 70 | 70 |
| 39 | 0.2 | 0 | 0 |
| 40 | 20.0 | 100 | 100 |
| 40 | 2.0 | 0 | 0 |
| Standard¹ | 20.0 | 100 | 100 |
| Standard¹ | 2.0 | 100 | 100 |
| Standard¹ | 0.2 | 0 | 100 |
| Control² | 0 | 0 | 0 |

| | | | |
|---|---|---|---|
| ¹Fipronil | | | |
| ²Untreated | | | |

Tables II, III and IV show considerable efficacy for the compounds of the invention. Those compounds displaying zero activity at all tested levels will need to be tested at higher doses to ascertain their effective concentrations.

## Claims

1. A compound of formula I 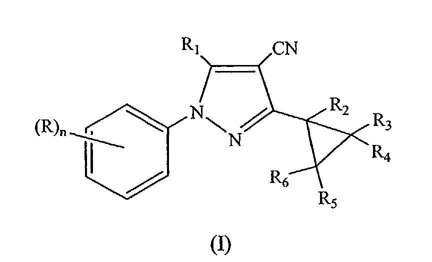 or a pharmaceutically acceptable salt thereof, wherein
R is halogen, OR₇, SOₘR₈, NO₂, CN, C₁-C₆haloalkyl or an optionally substituted C₁-C₆alkyl group;
n is 0 or an integer of 1, 2 or 3;
m is 0 or an integer of 1 or 2;
R₁ is H, halogen, NO₂, NR₉R₁₀, NR₁₁COR₁₂, NCHNR₉R₁₀ or NCHOR₁₃;
R₂, R₃, R₄, R₅ and R₆ are each independently H, halogen or a C₁-C₄alkyl, aryl or heteroaryl group each optionally substituted;
R₇ is H or a C₁-C₆alkyl, C₂-C₆alkenyl, C₂-C₆alkynyl, aryl or heteroaryl group each optionally substituted;
R₈ is a C₁-C₆alkyl, C₂-C₆alkenyl, C₂-C₆alkynyl, aryl or heteroaryl group, each optionally substituted;
R₉ and R₁₀ are each independently H, C₁-C₄haloalkyl or a C₁-C₆alkyl, C₂-C₆alkenyl, C₂-C₆alkynyl, aryl or heteroaryl group each optionally substituted or R₉ and R₁₀ may be taken together with the atom to which they are attached to form a 5- to 7-membered ring optionally containing 1 or 2 additional heteroatoms selected from O, N or S;
R₁₁ is H, COR₁₂ or an optionally substituted C₁-C₄alkyl group;
R₁₂ is a C₁-C₆alkyl, C₂-C₆alkenyl, C₂-C₆alkynyl, aryl or heteroaryl group each optionally substituted; and
R₁₃ is H or a C₁-C₆alkyl, aryl or heteroaryl group each optionally substituted;
or a stereoisomer or tautomer thereof.

2. The compound according to claim 1 wherein formula I has the proviso that R₃, R₄, R₅ and R₆ are not all -H, unless R₁ is halogen.

3. The compound according to claim 2 wherein R is halogen or haloalkyl.

4. The compound according to claim 2 wherein R₁ is H, halogen or NR₉R₁₀.

5. The compound according to claim 1 wherein R₅ and R₆ are H.

6. The compound according to claim 3 wherein R₂ is H, halogen, methyl or an optionally substituted phenyl group.

7. The compound according to claim 6 wherein R₁ is H or Cl.

8. The compound according to claim 7 wherein R is halogen or CF₃ and n is 3.

9. The compound according to claim 8 wherein R₂ is Cl or methyl and R₃ and R₄ are each independently H, Cl or Br.

10. The compound according to claim 2 wherein said compound is selected from the group consisting of:
5-chloro-3-(2,2-dichloro-1 methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-(2,4,6-trichlorophenyl)-1H-pyrazole-4-carbonitrile;
3-(2,2-dichloro-1,3-dimethylcyclopropyl)-1-[2,6-dichloro-4-(trifluoromethyl)phenyl]-1H-pyrazole-4-carbonitrile;
3-(2,2-dibromo-1-methylcyclopropyl)-1-[2,6-dichloro-4-(trifluoromethyl)phenyl]-1H-pyrazole-4-carbonitrile;
3-(2,2-dibromo-1-methylcyclopropyl)-1-(2,4,6-trichlorophenyl)-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
5-chloro-3-(2,2-dibromo-1-methylcyclopropyl)-1-[2,6-dichloro-4-(trifluoromethyl)phenyl-1 H-pyrazole-4-carbonitrile;
5-amino-3-(2,2-dibromo-1-methylcyclopropyl)-1-[2,6-dichloro-4-(trifluoromethyl)phenyl-1 H-pyrazole-4-carbonitrile;
5-bromo-3-(2,2-dibromo-1-methylcyclopropyl)-1-[2,6-dichloro-4-(trifluoromethyl)phenyl]-1H-pyrazole-4-carbonitrile;
5-amino-3-(2,2-dichloro-1-methylcyclopropyl)-1-(2,4,6-trichlorophenyl)-1H-pyrazole-4-carbonitrile;
5-chloro-3-cyclopropyl-1--[2,6-dichloro-4-(trifluoromethyl)phenyl-1 H-pyrazole-4-carbonitrile;
5-chloro-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-5-nitro-1 H-pyrazole-4-carbonitrile;
3-(2,2-dichloro-1-methylcyclopropyl)-1-[2,6-dichloro-4-(trifluoromethyl)phenyl]-5-iodo-1 H-pyrazole-4-carbonitrile;
3-(2,2-dichloro-1-methylcyclopropyl)-1-[2,6-dichloro-4-(trifluoromethyl)phenyl]-5-(dimethylamino)-1 H-pyrazole-4-carbonitrile;
3-(2,2-dichloro-1-methylcyclopyrazol)-1-[2,6-dichloro-4-(trifluoromethyl)phenyl]-5-(diethylamino)-1H-pyrazole-4-carbonitrile;
5-[(cyclopropanecarbonyl)amino-]-3-(2,2-dichloro-1 methylcyclopropyl)-1-[2,6-dichloro-4-(trifluoromethyl)phenyl]-1H-pyrazole-4-carbonitrile;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid methyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid propyl ester;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid ethyl ester;
the stereoisomers thereof; and the tautomers thereof; or a pharmaceutically acceptable salt thereof.

11. A method for the prevention, amelioration or control of ectoparasitic infection or infestation in a homeothermic animal which comprises providing to a homeothermic animal in need thereof a prophylactically, therapeutically or pharmaceutically effective amount of a compound of formula I according to claim 1.

12. The method according to claim 11 wherein the formula I has the proviso that R₃, R₄, R₅ and R₆ are not all -H, unless R₁ is halogen.

13. The method according to claim 12 wherein R is halogen or haloalkyl and n is 3.

14. The method according to claim 13 wherein R₅ and R₆ are H.

15. The method according to claim 14 wherein R₂ is H, halogen, methyl or an optionally substituted phenyl group.

16. The method according to claim 12 wherein the ectoparasite is selected from the group consisting of Diptera; Muscidae; Acarina; and Siphonáptera.

17. The method according to claim 16 wherein the ectoparasite is selected from the group consisting of fleas; ticks; lice; blow flies; face flies and horn flies.

18. The method according to claim 16 wherein the homeothermic animal is selected from the group consisting of cattle; sheep; horse; goat; pig; camel; water buffalo; donkey; rabbit; fallow deer; reindeer; mink; chinchilla; raccoon; chicken; geese; turkey; duck; dog and cat.

19. The method according to claim 17 wherein the homeothermic animal is selected from the group consisting of cattle, sheep, horse, dog, and cat.

20. A veterinary pour-on composition which comprises: a spreading oil; an aliphatic or aromatic hydrocarbon, mono or polyhydric alcohol, a C₁-C₁₀ alkyl ketone, or a mixture thereof; and an ectoparasiticidally effective amount of a compound of formula I according to claim 1.

21. The composition according to claim 20 wherein formula I has the proviso that R₃, R₄, R₅ and R₆ are not all -H, unless R₁ is halogen.

22. The composition according to claim 21 wherein R is halogen or haloalkyl and n is 3.

23. The composition according to claim 22 wherein said compound is selected from the group consisting of:
5-chloro-3-(2,2-dichloro-1 methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-(2,4,6-trichlorophenyl)-1H-pyrazole-4-carbonitrile;
3-(2,2-dichloro-1,3-dimethylcyclopropyl)-1-[2,6-dichloro-4-(trifluoromethyl)phenyl]-1H-pyrazole-4-carbonitrile;
3-(2,2-dibromo-1-methylcyclopropyl)-1-[2,6-dichloro-4-(trifluoromethyl)phenyl]-1H-pyrazole-4-carbonitrile;
3-(2,2-dibromo-1-methylcyclopropyl)-1-(2,4,6-trichlorophenyl)-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
5-chloro-3-(2,2-dibromo-1-methylcyclopropyl)-1-[2,6-dichloro-4-(trifluoromethyl)phenyl-1 H-pyrazole-4-carbonitrile;
5-amino-3-(2,2-dibromo-1-methylcyclopropyl)-1-[2,6-dichloro-4-(trifluoromethyl)phenyl-1 H-pyrazole-4-carbonitrile;
5-bromo-3-(2,2-dibromo-1-methylcyclopropyl)-1-[2,6-dichloro-4-(trifluoromethyl)phenyl]-1H-pyrazole-4-carbonitrile;
5-amino-3-(2,2-dichloro-1-methylcyclopropyl)-1-(2,4,6-trichlorophenyl)-1H-pyrazole-4-carbonitrile;
5-chloro-3-cyclopropyl-1--[2,6-dichloro-4-(trifluoromethyl)phenyl-1 H-pyrazole-4-carbonitrile;
5-chloro-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-[2, 6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-5-nitro-1 H-pyrazole-4-carbonitrile;
3-(2,2-dichloro-1-methylcyclopropyl)-1-[2,6-dichloro-4-(trifluoromethyl)phenyl]-5-iodo-1 H-pyrazole-4-carbonitrile;
3-(2,2-dichloro-1-methylcyclopropyl)-1-[2,6-dichloro-4-(trifluoromethyl)phenyl]-5-(dimethylamino)-1 H-pyrazole-4-carbonitrile;
3-(2,2-dichloro-1-methylcyclopyrazol)-1-[2,6-dichloro-4-(trifluoromethyl)phenyl]-5-(diethylamino)-1 H-pyrazole-4-carbonitrile;
5-[(cyclopropanecarbonyl)amino-]-3-(2,2-dichloro-1 methylcyclopropyl)-1-[2,6-dichloro-4-(trifluoromethyl)phenyl]-1H-pyrazole-4-carbonitrile;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid methyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid propyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid ethyl ester;
the stereoisomers thereof; and the tautomers thereof.

24. A veterinary pour-on composition which comprises: approximately 40-50% by weight xylene; approximately 20-30% by weight cyclohexanone; approximately 5-15% vegetable or mineral oil or a combination thereof; and approximately 10-25% of a compound selected from the group consisting of:
5-chloro-3-(2,2-dichloro-1 methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-(2,4,6-trichlorophenyl)-1H-pyrazole-4-carbonitrile;
3-(2,2-dichloro-1,3-dimethylcyclopropyl)-1-[2,6-dichloro-4-(trifluoromethyl)phenyl]-1H-pyrazole-4-carbonitrile;
3-(2,2-dibromo-1-methylcyclopropyl)-1-[2,6-dichloro-4-(trifluoromethyl)phenyl]-1H-pyrazole-4-carbonitrile;
3-(2,2-dibromo-1-methylcyclopropyl)-1-(2,4,6-trichlorophenyl)-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
5-chloro-3-(2,2-dibromo-1-methylcyclopropyl)-1-[2,6-dichloro-4-(trifluoromethyl)phenyl-1 H-pyrazole-4-carbonitrile;
5-amino-3-(2,2-dibromo-1-methylcyclopropyl)-1-[2,6-dichloro-4-(trifluoromethyl)phenyl-1 H-pyrazole-4-carbonitrile;
5-bromo-3-(2,2-dibromo-1-methylcyclopropyl)-1-[2,6-dichloro-4-(trifluoromethyl)phenyl]-1H-pyrazole-4-carbonitrile;
5-amino-3-(2,2-dichloro-1-methylcyclopropyl)-1-(2,4,6-trichlorophenyl)-1H-pyrazole-4-carbonitrile;
5-chloro-3-cyclopropyl-1--[2,6-dichloro-4-(trifluoromethyl)phenyl-1H-pyrazole-4-carbonitrile;
5-chloro-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-5-nitro-1 H-pyrazole-4-carbonitrile;
3-(2,2-dichloro-1-methylcyclopropyl)-1-[2,6-dichloro-4-(trifluoromethyl)phenyl]-5-iodo-1 H-pyrazole-4-carbonitrile;
3-(2,2-dichloro-1-methylcyclopropyl)-1-[2,6-dichloro-4-(trifluoromethyl)phenyl]-5-(dimethylamino)-1 H-pyrazole-4-carbonitrile;
3-(2,2-dichloro-1-methylcyclopyrazol)-1-[2,6-dichloro-4-(trifluoromethyl)phenyl]-5-(diethylamino)-1 H-pyrazole-4-carbonitrile;
5-[(cyclopropanecarbonyl)amino-]-3-(2,2-dichloro-1methylcyclopropyl)-1-[2,6-dichloro-4-(trifluoromethyl)phenyl]-1H-pyrazole-4-carbonitrile;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid methyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid propyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid ethyl ester;
the stereoisomers thereof; and the tautomers thereof.

25. The composition according to claim 24 wherein an effective dosage of said compound is within the range of about 0.1 mg/kg to 100 mg/kg of animal body weight.

26. A veterinary composition which comprises a pharmaceutically acceptable carrier and about 0.1 ppm to 5000 ppm of a compound selected from the group consisting of:
5-chloro-3-(2,2-dichloro-1 methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-(2,4,6-trichlorophenyl)-1H-pyrazole-4-carbonitrile;
3-(2,2-dichloro-1,3-dimethylcyclopropyl)-1-[2,6-dichloro-4-(trifluoromethyl)phenyl]-1H-pyrazole-4-carbonitrile;
3-(2,2-dibromo-1-methylcyclopropyl)-1-[2,6-dichloro-4-(trifluoromethyl)phenyl]-1H-pyrazole-4-carbonitrile;
3-(2,2-dibromo-1-methylcyclopropyl)-1-(2,4,6-trichlorophenyl)-1H-pyrazole-4-carbonitrile;
3-(2,2-dichloro-1-methyleyclopropyl)-1-[2,6-dichloro-4-(trifluoromethyl)phenyl]-1H-pyrazole-4-carbonitrile;
5-chloro-3-(2,2-dibromo-1-methylcyclopropyl)-1-[2,6-dichloro-4-(trifluoromethyl)phenyl-1H-pyrazole-4-carbonitrile;
5-amino-3-(2,2-dibromo-1-methylcyclopropyl)-1-[2,6-dichloro-4-(trifluoromethyl)phenyl-1 H-pyrazole-4-carbonitrile;
5-bromo-3-(2,2-dibromo-1-methylcyclopropyl)-1-[2,6-dichloro-4-(trifluoromethyl)phenyl]-1H-pyrazole-4-carbonitrile;
5-amino-3-(2,2-dichloro-1-methylcyclopropyl)-1-(2,4,6-trichlorophenyl)-1H-pyrazole-4-carbonitrile;
5-chloro-3-cyclopropyl-1--[2,6-dichloro-4-(trifluoromethyl)phenyl-1H-pyrazole-4-carbonitrile;
5-chloro-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-(2,4,6-trichlorophenyl)-1H-pyrazole-4-carbonitrile;
5-bromo-3-(2,2-dichloro-1-methylcyclopropyl)-1-[2,6-dichloro-4-(trifluoromethyl)phenyl]-1H-pyrazole-4-carbonitrile;
3-(2,2-dichloro-1-methylcyclopropyl)-1-[2,6-dichloro-4-(trifluoromethyl)phenyl]-5-nitro-1 H-pyrazole-4-carbonitrile;
3-(2,2-dichloro-1-methylcyclopropyl)-1-[2,6-dichloro-4-(trifluoromethyl)phenyl]-5-iodo-1 H-pyrazole-4-carbonitrile;
3-(2,2-dichloro-1-methylcyclopropyl)-1-[2,6-dichloro-4-(trifluoromethyl)phenyl]-5-(dimethylamino)-1H-pyrazole-4-carbonitrile;
3-(2,2-dichloro-1-methylcyclopyrazol)-1-[2,6-dichloro-4-(trifluoromethyl)phenyl]-5-(diethylamino)-1 H-pyrazole-4-carbonitrile;
5-[(cyclopropanecarbonyl)amino-]-3-(2,2-dichloro-1methylcyclopropyl)-1-[2,6-dichloro-4-(trifluoromethyl)phenyl]-1H-pyrazole-4-carbonitrile;
N-{4-cyano-5-(2,2-dichloro-1-methyl-cyclopropyl)-2-[2,6-dichloro-4-(trifluoromethyl)-phenyl]-2H-pyrazol-3-yl}-formimidic acid methyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid propyl ester;
N-{4-cyano-5-(2,2-dichloro-1-methyl-cyclopropyl)-2-[2,6-dichloro-4-(trifluoromethyl)-phenyl]-2-H-pyrazol-3-yl}-formimidic acid ethyl ester;
the stereoisomers thereof; and the tautomers thereof.

27. The composition according to claim 26 which comprises about 0.5 ppm to 1000 ppm of said compound.

28. The composition according to claim 27 which comprises about 0.2 ppm to 20 ppm of said compound.

29. A composition for the prevention, amelioration or control of external parasites on animals and humans comprising a pharmaceutically acceptable carrier and an ectoparasiticidally effective amount of a compound as claimed in claim 1.
